# EUROPEAN PATENT SPECIFICATION

(11) **EP 1 082 941 B1**
(45) Date of publication and mention of the grant of the patent: **23.03.2005**
(21) Application number: 99202972.8
(22) Date of filing: 10.09.1999
(51) Int. Cl.: A61B 17/064

(54) **Tissue repair device**
Bindeelement zur Wundheilung
Elément de fermeture d'une plaie

(43) Date of publication of application: 14.03.2001
(73) Proprietor: Zimmer Technology, Inc., Chicago, Illinois 60606 (US)
(72) Inventor: Lin, Steve T., Fort Wayne, IN 46804 (US); Krebs, Steven L., Arlington, TX 76017 (US)
(74) Representative: Mays, Julie

(56) References cited:
- EP-A- 0 632 999
- DE-A- 4 210 801
- US-A- 5 059 206
- US-A- 5 980 524

## Description

### FIELD OF THE INVENTION

The present invention relates generally to the orthopedic surgical arts and more specifically to absorbable polymeric devices used to repair tissues, especially torn meniscal cartilage.

### BACKGROUND OF THE INVENTION

Surgery to the human body often requires the attachment of tissues adjacent one another such as in the repair of the knee. The human knee joint contains a pair of cartilage pads which function to absorb shock and prevent friction between the opposing bones in the joint. Each pad is called a meniscus. The two pads are referred to as the meniscal cartilage. One or both of these pads can become torn causing pain and debilitation. If these tears are not treated, further deterioration of the joint can occur. Past medical treatment involved excision of the torn cartilage, sometimes including complete removal of the meniscus. Complete removal of the meniscus has been shown to cause degenerative changes in the joint, often resulting in a joint replacement. Modern surgical treatment focuses on repair of the tissue rather than excision. The most popular method of treatment involves the use of arthoscopic means to access the joint. The devices used to repair the cartilage range from sutures to staples and tacks such as taught in U.S Patent 5,059,206. Requirements that are common to all of these devices are the need for the device to hold the torn tissue in close apposition and for the device to resorb after the tissues have healed Regarding the first requirement, sutures can be tightened to hold the tissues close together when first applied but the tension is variable between each stitch and the sutures will loosen with time. Staples can be difficult to place and once placed there is no ability to adjust the compression between the opposing surfaces of the torn cartilage. Regarding the second requirement, resorbtion, or dissolution over time, of the device removes the need for later removal of the device and also removes the possibility of the device interfering with the normal functioning of the cartilage if the device was not removed.

### SUMMARY OF THE INVENTION

The present invention solves the problems of the prior art by providing a tissue repair device composed of a drawn biodegradable polymer. The repair device makes use of molecular orientation, residual stress, and T_{g} depression to provide a compressive force across torn tissue. The repair device of the present invention includes barbs or hooks at either end of the device to hold it firmly in the tissue while the force is applied.

### DESCRIPTION OF THE DRAWINGS

Figure 1 is a side view of finished repair device according to the present invention.
Figure 2 is a side view of the repair device of Fig. 1 prior to the drawing operation.
Figure 3 is a side view of the repair device of Fig. 1 after exposure to an aqueous environment
Figure 4 is a top view of the repair device of Fig. 1 just after insertion into tissue and before exposure to water has caused contraction of the polymer.
Figure 5 is a top view of the repair device of Fig. 1 after insertion into tissue and after exposure to water has caused contraction of the device.

### DETAILED DESCRIPTION OF THE INVENTION

The following descriptions of the preferred embodiment are not intended to be exhaustive or to limit the invention to the precise forms disclosed rather they are chosen in order to explain the invention so that one skilled in the art might utilize their teachings.

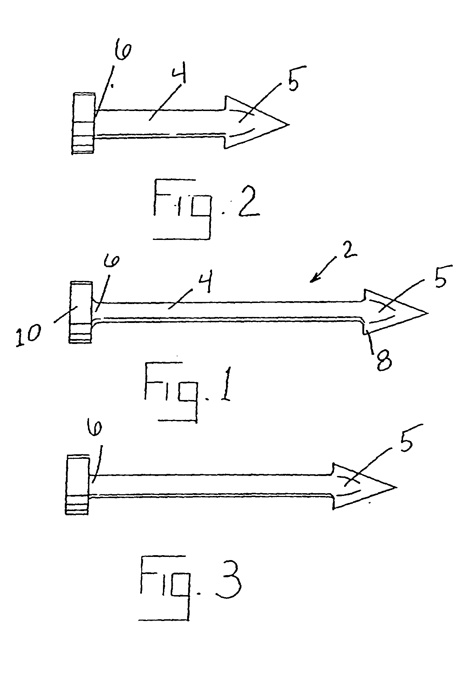

Figure 1 illustrates one embodiment of the invention. The device 2 comprises a longitudinally oriented bioresorbable polymer shaft 4, having two ends 5 and 6. The first end 5 of the shaft has a tapered point for penetrating tissue. Nearby the first end are a plurality of gripping means 8, such as barbs, for gripping tissue. The gripping means allow for movement in one direction. The second end 6 terminates in an end cap 10 for applying a compressive force to tissue and adapted for receiving a driver. The shaft 4 is smooth and separates the first and second ends 5 and 6.

The repair device is first molded of a bioresorbable material to form the as-molded device shown in Fig. 2. After molding the shaft 4 is drawn, or stretched, to align the polymer molecules. Drawing the polymer aligns the molecules and leaves the molecules in a state of residual stress. The diameter of the shaft 4 is reduced and it is lengthened after drawing.

The residual stress in the device is a key component of the action of the device. When an absorbable polymer is placed in an aqueous environment the water acts as a solvent for the amorphous regions of the polymer, lowering the glass transition temperature of the polymer. The glass transition temperature is the temperature at which molecular motion increases to the point of allowing the polymer to become more flexible. Since the polymer molecules in the shaft 4 of the device are stretched and under stress prior to exposure to an aqueous environment they will shrink back to a percentage of their original length when exposed to water. This shrinkage pulls the first end 5 and second end 6 of the device towards one another as shown in Fig. 3. The end cap 10 holds the second end 6 in place relative to adjacent tissues. The barbs 8 on the first end 5 of the device hold the first end of the device relative to adjacent tissue. Since the first end 10 and second end 11 of the device are firmly fixed in the tissue adjacent each end, the shrinking central portion applies a compressive force that pushes the tissues together. As the tissue heals, the pressure on the tissue is released by the polymer resorbable.

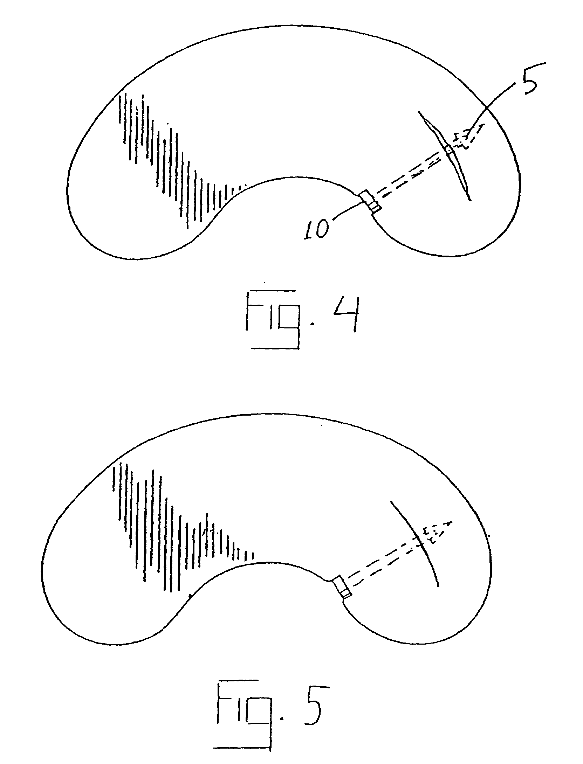

Figure 4 illustrates the device as implanted in a meniscal cartilage. A driver is used to press against the end cap 10 so that the first end 5 of the device is inserted into the meniscal cartilage, penetrating the cartilage. The device is advanced through the cartilage passing across the tear and penetrating to the opposite side of the tear. Once the device is placed in the cartilage, the driver is removed. Figure 5 illustrates the action of the device after sufficient exposure to water. The device will contract and draw the barbs 8 and end cap 10 toward one another thereby pulling the torn tissue surfaces together. It should be understood that the invention is not to be limited to the precise forms disclosed rather, they may be modified within the scope of the appended claims.

## Claims

1. A tissue repair device composed of a bioresorbable polymer (2), comprising a shaft (4) including a longitudinal axis and first and second ends (5,6) spaced along the axis, each of the first and second ends (5,6) including gripping means (8) for gripping tissue, **characterised in that** the shaft (4) is responsive to exposure in an aqueous environment to shrink and compress tissue along the longitudinal axis upon exposure of the device (2) to an aqueous environment.

2. The tissue repair device (2) of Claim 1 **characterised in that** the first end (5) further includes a tapered tip for penetrating tissue.

3. The tissue repair device (2) of Claim 1 or Claim 2 **characterised in that** the gripping means (8) includes barbs extending from the shaft (4).

4. The tissue repair device (2) of any preceding claim **characterised in that** the second end (6) is adapted to be driven by a driver.

5. The tissue repair device (2) of any preceding claim **characterised in that** the shaft (4) includes a bioresorbable polymer having residual stresses imparted into it and preserved in it along the longitudinal axis, the resorbable polymer being responsive to exposure in an aqueous environment to shrink along the longitudinal axis.

6. The tissue repair device (2) of any preceding claim **characterised in that** the shaft comprises a drawn, oriented polymer.

7. The tissue repair device (2) of Claim 5 or Claim 6 **characterised in that** the shaft (4), on continued exposure to an aqueous environment, resorbs and eases tissue compression.

8. A method for fabricating a tissue repair device (2) from a bioresorbable polymer comprising first forming a device having a shaft (4) including a longitudinal axis and first and second ends (5,6) spaced along the axis, each of the first and second ends including gripping means (8) for gripping tissues; and then stretching the shaft (4) to impart residual stresses in it, so that it is responsive to exposure in an aqueous environment to shrink and compress tissue along the longitudinal axis upon exposure of the device (2) to an aqueous environment.

9. The method of Claim 8 wherein the forming step is a molding operation.

## Patentansprüche

1. Gewebebindeelement (2), das ein biologisch resorbierbares Polymer aufweist, mit einem Schaft (4) mit einer Längsachse und einem ersten und zweiten Ende (5, 6), die entlang der Achse beabstandet sind, wobei sowohl das erste als auch zweite Ende (5, 6), eine Greifeinrichtung (8) zum Ergreifen des Gewebes aufweisen, **dadurch gekennzeichnet, daß** der Schaft (4) auf die Einwirkung einer wäßrigen Umgebung so reagiert, daß bei Einwirkung einer wäßrigen Umgebung auf das Element (2) entlang der Längsachse schrumpft und Gewebe komprimiert wird.

2. Gewebebindeelement (2) nach Anspruch 1, **dadurch gekennzeichnet, daß** das erste Ende (5) ferner eine kegelförmige Spitze zum Durchdringen von Gewebe aufweist.

3. Gewebebindeelement (2) nach Anspruch 1 oder 2, **dadurch gekennzeichnet, daß** die Greifeinrichtung (8) Widerhaken aufweist, die sich vom Schaft (4) erstrecken.

4. Gewebebindeelement (2) nach einem der vorhergehenden Ansprüche, **dadurch gekennzeichnet, daß** das zweite Ende (6) geeignet ist, von einem Treibelement getrieben zu werden.

5. Gewebebindeelement (2) nach einem der vorhergehenden Ansprüche, **dadurch gekennzeichnet, daß** der Schaft (4) ein biologisch resorbierbares Polymer mit Restspannungen aufweist, die ihm verliehen sind und in ihm entlang der Längsachse erhalten bleiben, wobei das abbaubare Polymer auf die Einwirkung einer wäßrigen Umgebung so reagiert, daß es entlang der Längsachse schrumpft.

6. Gewebebindeelement (2) nach einem der vorhergehenden Ansprüche, **dadurch gekennzeichnet, daß** der Schaft ein gezogenes, orientiertes Polymer aufweist.

7. Gewebebindeelement (2) nach Anspruch 5 oder 6, **dadurch gekennzeichnet, daß** der Schaft (4) bei fortgesetzter Einwirkung einer wäßrigen Umgebung resorbiert wird und den Gewebekompressionsdruck abbaut.

8. Verfahren zur Herstellung eines Gewebebindeelements (2) aus einem biologisch resorbierbares Polymer, mit den Schritten: zuerst erfolgendes Ausbilden eines Elements mit einem Schaft (4) mit einer Längsachse und einem ersten und zweiten Ende (5, 6), die entlang der Achse beabstandet sind, wobei sowohl das erste als auch das zweite Ende Greifeinrichtungen (8) zum Ergreifen von Gewebe aufweisen; und anschließendes Strecken des Schafts (4), um ihm Restspannungen zu verleihen, so daß er auf die Einwirkung einer wäßrigen Umgebung so reagiert, daß er bei Einwirkung einer wäßrigen Umgebung auf das Element (2) entlang der Längsachse schrumpft und Gewebe komprimiert wird.

9. Verfahren nach Anspruch 8, wobei der Ausbildungsschritt ein Formvorgang ist.

## Revendications

1. Dispositif de réparation d'un tissu composé d'un polymère biorésorbable (2), comprenant une tige (4) incluant un axe longitudinal et des première et seconde extrémités (5, 6) espacées le long de l'axe, chacune des première et seconde extrémités (5, 6) incluant des moyens de préhension (8) pour saisir le tissu, **caractérisé en ce que** la tige (4) est sensible à une exposition dans un environnement aqueux pour se resserrer et comprimer le tissu le long de l'axe longitudinal lors de l'exposition du dispositif (2) à un environnement aqueux.

2. Dispositif de réparation d'un tissu (2) selon la revendication 1 **caractérisé en ce que** la première extrémité (5) inclut de plus un bout pointu pour pénétrer dans le tissu.

3. Dispositif de réparation d'un tissu (2) selon la revendication 1 ou la revendication 2 **caractérisé en ce que** les moyens de préhension (8) incluent des crochets s'étendant à partir de la tige (4).

4. Dispositif de réparation d'un tissu (2) selon l'une quelconque des revendications précédentes **caractérisé en ce que** la seconde extrémité (6) est adaptée pour être guidée par un conducteur.

5. Dispositif de réparation d'un tissu (2) selon l'une quelconque des revendications précédentes **caractérisé en ce que** la tige (4) inclut un polymère biorésorbable ayant des tensions résiduelles transmises dans celui-ci et conservées dans celui-ci le long de l'axe longitudinal, le polymère résorbable étant sensible à une exposition dans un environnement aqueux pour se resserrer le long de l'axe longitudinal.

6. Dispositif de réparation d'un tissu (2) selon l'une quelconque des revendications précédentes **caractérisé en ce que** la tige comprend un polymère étiré orienté.

7. Dispositif de réparation d'un tissu (2) selon la revendication 5 ou la revendication 6 **caractérisé en ce que** la tige (4), sous exposition continue à un environnement aqueux, se résorbe et facilite la compression du tissu.

8. Procédé destiné à fabriquer un dispositif de réparation d'un tissu (2) à partir d'un polymère biorésorbable comprenant de former d'abord un dispositif ayant une tige (4) incluant un axe longitudinal et des première et secondes extrémités (5, 6) espacées le long de l'axe, chacune des première et seconde extrémités (5, 6) incluant des moyens de préhension (8) pour saisir le tissu ; et ensuite d'étirer la tige (4) pour transmettre les tensions résiduelles dans celui-ci, de sorte qu'il soit sensible à une exposition dans un environnement aqueux pour se resserrer et comprimer le tissu le long de l'axe longitudinal lors de l'exposition du dispositif (2) à un environnement aqueux.

9. Procédé selon la revendication 8 dans lequel l'étape de formation est une opération de moulage.
